# EUROPÄISCHE PATENTSCHRIFT

(11) **EP 1 382 315 B1**
(45) Veröffentlichungstag und Bekanntmachung des Hinweises auf die Patenterteilung: **17.09.2008**
(21) Anmeldenummer: 03014403.4
(22) Anmeldetag: 27.06.2003
(51) Int. Cl.: A61F 2/44

(54) **Zwischenwirbelimplantat**
Intervertebral implant
Prothèse intervertébrale

(30) Priorität: 16.07.2002 DE 10232116
(43) Veröffentlichungstag der Anmeldung: 21.01.2004
(73) Patentinhaber: Aesculap AG, 78532 Tuttlingen (DE)
(72) Erfinder: Bronsard, Jean-Jacques, 13007 Marseille (FR); Schneid. Susanne, 78532 Tuttlingen (DE)
(74) Vertreter: Hoeger, Stellrecht & Partner Patentanwälte

(56) Entgegenhaltungen:
- DE-U- 20 104 863
- FR-A- 2 771 282
- US-A1- 2002 045 943
- US-A1- 2002 052 656

## Beschreibung

Die Erfindung betrifft ein Zwischenwirbelimplantat mit an benachbarten Wirbelkörpern anlegbaren Stützflächen, die durch die Außenseiten von zwei gegeneinander schwenkbaren Schenkeln des Zwischenwirbelimplantates gebildet werden, und mit einem zwischen den Schenkeln drehbar angeordneten Spreizkörper, der bei Verdrehung die Schenkel mittels einer Anlagefläche unterschiedlich weit aufspreizt, wobei die Anlagefläche mindestens einen radialen Vorsprung aufweist, durch den bei Verdrehung des Spreizkörpers die Schenkel weiter aufgespreizt werden als in den beiden in Umfangsrichtung danebenliegenden Bereichen, wobei der Spreizkörper in das Zwischenwirbelimplantat einschraubbar ist.

Ein Zwischenwirbelimplantat mit an benachbarten Wirbelkörper anlegbaren Stützflächen, die durch die Außenseiten von zwei gegeneinander schwenkbaren Schenkeln des Zwischenwirbelimplantates gebildet werden, und mit einem zwischen den Schenkeln drehbar angeordneten Spreizkörper, der bei Verdrehung der Schenkel mittels einer Anlagefläche unterschiedlich weit aufspreizt, ist beispielsweise aus der DE 44 16 605 C1 oder der FR 2 771 282 A1 bekannt. Dort wird der Spreizkörper zwischen die beiden Schenkel eingeschraubt und spreizt sie bei zunehmender Einschraubtiefe zunehmend auseinander. Die Anlagefläche des Spreizkörpers ist dabei konisch ausgebildet und liegt an den Innenflächen der Schenkel an. Der eingeschraubte Spreizkörper muß bei dieser Konstruktion dagegen gesichert werden, daß er sich unbeabsichtigt in seiner Winkelstellung verstellt, denn diese Verstellung würde zu einer Veränderung des Abstandes der beiden Stützflächen führen. Um dies zu vermeiden, müssen gesonderte Maßnahmen zur Festlegung des Spreizkörpers gegen unbeabsichtigte Verdrehung getroffen werden. Des Weiteren ist ein Zwischenwirbelimplantat gemäß dem Oberbegriff des Anspruchs 1 aus der US 2002/052656 A1 bekannt.

Es ist Aufgabe der Erfindung, ein gattungsgemäßes Zwischenwirbelimplantat so auszubilden, daß eine unbeabsichtigte Verdrehung des Spreizkörpers auch verhindert wird, ohne daß besondere Feststellmaßnahmen für den Spreizkörper notwendig werden.

Diese Aufgabe wird bei einem Zwischenwirbelimplantat der eingangs beschriebenen Art erfindungsgemäß dadurch gelöst, daß über den Umfang des Spreizkörpers mehrere Vorsprünge verteilt sind, so daß ein Mehrkantquerschnitt entsteht, und daß der Spreizkörper in eine in am Zwischenwirbelimplantat angeordnete Innengewindebohrung einschraubbar ist.

Dadurch wird beim Verdrehen des Spreizkörpers der Abstand zwischen den Schenkeln periodisch vergrößert und verkleinert, verstellt man den Spreizkörper so, daß die Schenkel an den neben einem solchen Vorsprung liegenden Bereichen anliegen, kann ein unbeabsichtigtes Verdrehen des Spreizkörpers nicht mehr erfolgen, da dieses zwangsläufig zu einer weiteren Aufspreizung der Schenkel führen würde, und diese Aufspreizung wird durch die von außen gegen die Schenkel drückenden Wirbelkörper verhindert. Der Spreizkörper gelangt also mit anderen Worten über einen Totpunkt in seine endgültige Winkelstellung und verbleibt in dieser, da er auch nur über diesen Totpunkt wieder in eine andere Winkelstellung gelangen könnte. Der Übergang über einen solchen Totpunkt ist jedoch nur dann möglich, wenn ein erhöhtes Drehmoment auf den Spreizkörper ausgeübt wird, und dies kann nur durch ein bewußt angesetztes Werkzeug erfolgen und nicht unbeabsichtigt. Durch die unterschiedliche Einschraubtiefe drückt der Spreizkörper die Schenkel unterschiedlich weit auseinander. Besonders günstig ist es, daß über den Umfang des Spreizkörpers mehrere Vorsprünge verteilt sind, beispielsweise drei oder vier derartige Vorsprünge, so daß ein Mehrkantquerschnitt entsteht.

Besonders günstig ist es, wenn der Vorsprung durch eine Kante zwischen zwei benachbarten Umfangsbereichen des Spreizkörpers gebildet wird.

Diese Kante kann insbesondere im wesentlichen parallel zur Drehachse des Spreizkörpers verlaufen.

Die Umfangsflächen zwischen den Kanten können im wesentlichen eben sein, es ist auch möglich, die Umfangsflächen in Richtung der Drehachse des Spreizkörpers ballig ausgebaucht zu gestalten.

Insbesondere können die Kanten abgerundet sein.

Bei dem Spreizkörper kann es sich in der beschriebenen Weise um einen Spreizkörper handeln, der in das Zwischenwirbelimplantat einschraubbar ist und durch die unterschiedliche Einschraubtiefe die Schenkel unterschiedlich weit auseinanderdrückt. Es wäre aber auch möglich, eine exzentrische Anlagefläche zu verwenden, die durch die Vorsprünge gerastert ist, so daß jeder Winkelstellung durch die Exzentrizität die Anlagefläche eine andere Aufspreizung entspricht. Trotzdem wird eine ungewollte Winkelverstellung des Spreizkörpers nach beiden Richtungen durch die radialen Vorsprünge verhindert.

Die nachfolgende Beschreibung bevorzugter Ausführungsformen der Erfindung dient im Zusammenhang mit der Zeichnung der näheren Erläuterung. Es zeigen:

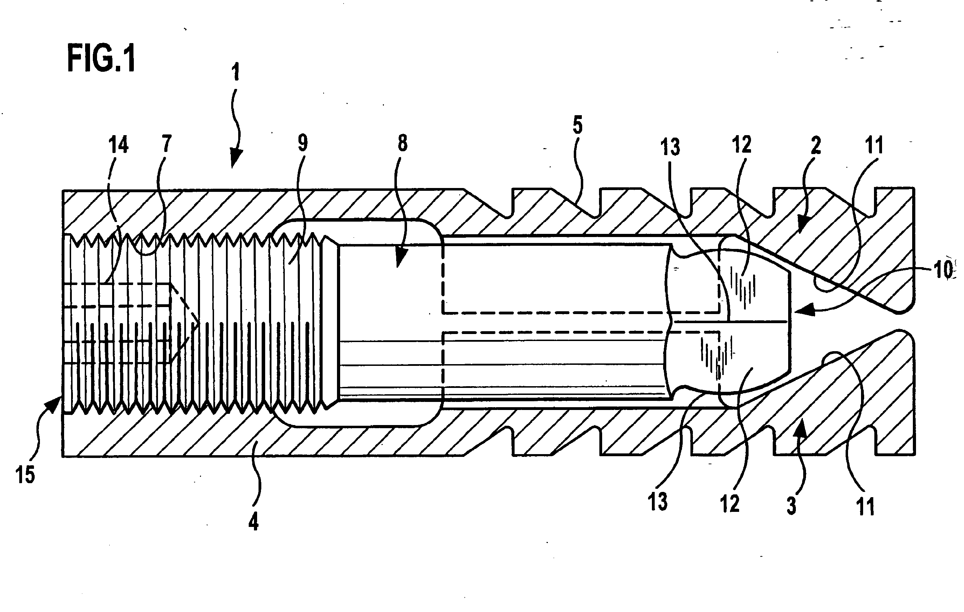

- Figur 1:: einen Längsschnitt durch ein Zwischenwirbelimplantat mit einem in diesen einschraubbaren Spreizkörper;
- Figur 2:: eine Ansicht ähnlich Figur 1 mit dem Spreizkörper in einer Winkelstellung, bei der ein radialer Vorsprung die Schenkel des Zwischenwirbelimplantates aufspreizt;
- Figur 3:: eine Ansicht des Zwischenwirbelimplantates der Figur 2 von der Vorderseite her;
- Figur 4:: eine Ansicht ähnlich Figur 2 mit dem Spreizkörper in einer Winkelstellung, in der die Umfangsflächen neben einem radialen Vorsprung an den Schenkeln des Zwischenwirbelimplantates anliegen, also in einer stabilen Lage und
- Figur 5:: eine Ansicht des Zwischenwirbelimplantates der Figur 4 von der Vorderseite her.

Das in der Zeichnung dargestellte Zwischenwirbelimplantat umfaßt einen U-förmigen Stützkörper 1 mit zwei parallelen Schenkeln 2, 3, die über einen Steg 4 miteinander verbunden und zu ihren freien Enden hin elastisch aufbiegbar sind, d. h. die Schenkel können auseinandergespreizt werden. Beide Schenkel 2, 3 weisen auf der Außenseite eine Profilierung 5 auf, diese Außenseite bildet eine Stützfläche aus, die beim Einschieben des Zwischenwirbelimplantates in den Zwischenraum zwischen benachbarten Wirbeln 6 an diesen zur Anlage kommt.

Im Steg 4 ist parallel zu den Schenkeln 2, 3 verlaufend eine Innengewindebohrung 7 angeordnet, in die ein Spreizstift 8 eingeschraubt ist, dessen einem Einschraubgewinde 9 gegenüberliegendes Ende 10 an keilförmig nach innen gerichteten Flächen 11 der beiden Schenkel 2, 3 anliegt. Durch unterschiedlich tiefes Einschrauben des Spreizstiftes 8 in die Innengewindebohrung 7 treibt das freie Ende 10 des Spreizstiftes 8 die Flächen 11 mehr oder weniger auseinander und spreizt dadurch die Schenkel 2, 3 mehr oder weniger weit auf.

Im Bereich des freien Endes 10 ist der Spreizstift 8 als Vierkant ausgebildet mit vier um 90° gegeneinander gedrehten Außenflächen 12, die in Richtung der Längsachse des Spreizstiftes 8 geringfügig ballig nach außen ausgebaucht sind, die aber quer dazu eben ausgebildet sind. Benachbarte Außenflächen 12 gehen längs einer Kante 13 ineinander über, die sich als Schnittlinie der beiden aneinander angrenzenden Außenflächen 12 ergibt und die geringfügig abgerundet ist. Jede der vier Kanten 13 bildet somit einen radialen Vorsprung im Bereich des freien Endes 10, die Kanten 13 haben den größten radialen Abstand von der Drehachse des Spreizstiftes 8, der radiale Abstand der Außenflächen 12 ist in deren gesamtem Umfangsbereich kleiner als der radiale Abstand der Kanten 13.

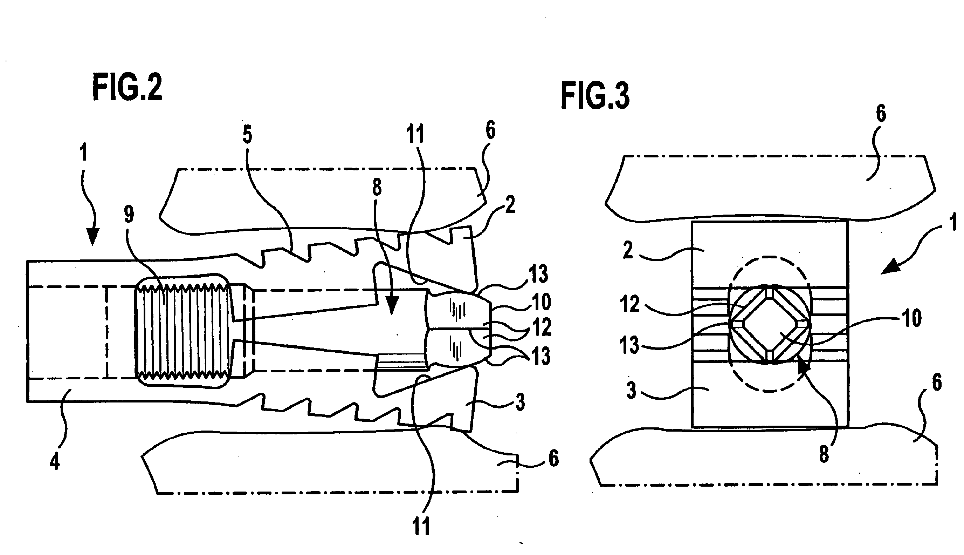

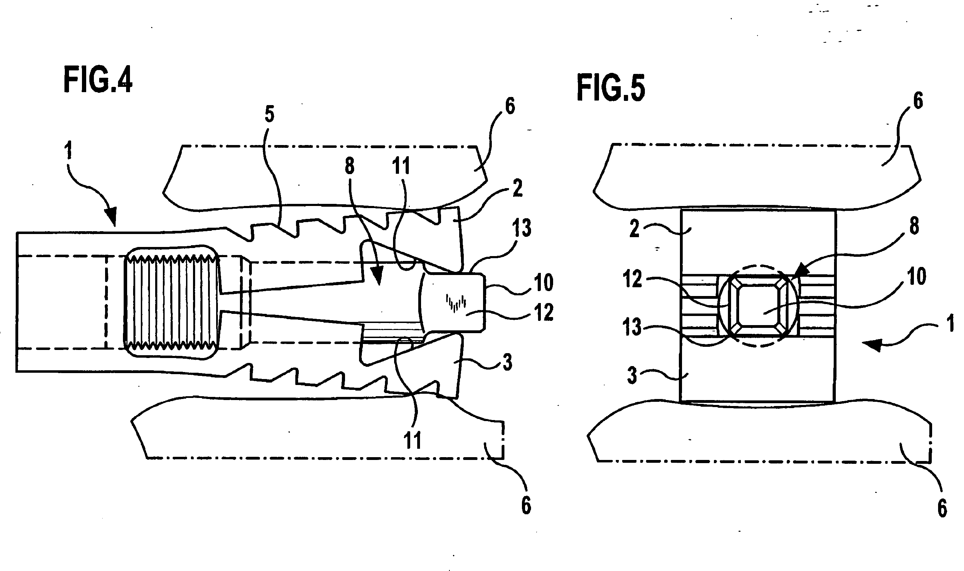

Wenn der Spreizstift 8 mit Hilfe eines Werkzeuges verdreht wird, welches in eine entsprechende Sacklochbohrung 14 an dem dem freien Ende 10 gegenüberliegenden Ende 15 im Spreizstift 8 eingreifen kann, dann gleiten abwechselnd Kanten 13 und Außenflächen 12 an den Flächen 11 entlang, so daß die Flächen 11 dadurch periodisch auseinander- und wieder zusammengeschwenkt werden. Immer dann, wenn eine Kante 13 an einer Fläche 11 anliegt, erhält man eine maximale Aufspreizung der Schenkel 2, 3 (dies ist in den Figuren 2 und 3 dargestellt), immer dann, wenn die Außenflächen 12 des Spreizstiftes 8 an den Flächen 11 anliegen, ergibt sich eine minimale Aufspreizung (Figuren 4 und 5). Durch das Einschrauben des Spreizstiftes 8 wird dabei bei zunehmender Einschraubtiefe insgesamt eine zunehmende Aufspreizung der Schenkel erreicht. Wesentlich ist dabei, daß beim Verdrehen des Spreizstiftes 8 aus einer Stellung, wie sie in den Figuren 4 und 5 dargestellt ist, bei der also eine Außenfläche 12 an einer Fläche 11 anliegt, in beiden Richtungen eine Aufweitung der Schenkel 2, 3 notwendig ist, und dies kann nur mit Hilfe des Werkzeuges unter Aufbringen eines relativ großen Momentes erfolgen, da die Schenkel 2, 3 durch die an den Außenseiten der Schenkel 2, 3 anliegenden Wirbelkörper 6 gegeneinander gepreßt werden, zum Verdrehen muß dieser Druck überwunden werden, der von den Wirbelkörpern 6 ausgeübt wird. Dies ist mit einem Werkzeug möglich, aber nicht ohne dieses und ungewollt. Dadurch ergibt sich eine Drehsicherung des Spreizstiftes 8 in jeder Stellung, in der eine Außenfläche 12 an einer Fläche 11 anliegt.

## Patentansprüche

1. Zwischenwirbelimplantat mit an benachbarten Wirbelkörpern (6) anlegbaren Stützflächen, die durch die Außenseiten von zwei gegeneinander schwenkbaren Schenkeln (2, 3) des Zwischenwirbelimplantates gebildet werden, und mit einem zwischen den Schenkeln (2, 3) drehbar angeordneten Spreizkörper (8), der bei Verdrehung die Schenkel (2, 3) mittels einer Anlagefläche unterschiedlich weit aufspreizt, wobei die Anlagefläche mindestens einen radialen Vorsprung (13) aufweist, durch den bei Verdrehung des Spreizkörpers (8) die Schenkel (2, 3) weiter aufgespreizt werden als in den beiden in Umfangsrichtung danebenliegenden Bereichen (12), wobei der Spreizkörper (8) in das Zwischenwirbelimplantat einschraubbar ist, **dadurch gekennzeichnet, daß** über den Umfang des Spreizkörpers (8) mehrere Vorsprünge (13) verteilt sind, so daß ein Mehrkantquerschnitt entsteht, und daß der Spreizkörper (8) in eine am Zwischenwirbelimplantat angeordnete Innengewindebohrung (7) einschraubbar ist.

2. Implantat nach Anspruch 1, **dadurch gekennzeichnet, daß** der Vorsprung (13) durch eine Kante zwischen zwei benachbarten Umfangsbereichen (12) des Spreizkörpers (8) gebildet wird.

3. Implantat nach Anspruch 2, **dadurch gekennzeichnet, daß** der Vorsprung (13) im wesentlichen parallel zur Drehachse des Spreizkörpers (8) verläuft.

4. Implantat nach einem der voranstehenden Ansprüche, **dadurch gekennzeichnet, daß** die Umfangsflächen (12) zwischen den Vorsprüngen (13) im wesentlichen eben sind.

5. Implantat nach einem der Ansprüche 1 bis 3, **dadurch gekennzeichnet, daß** die Umfangsflächen (12) in Richtung der Drehachse des Spreizkörpers (8) ballig ausgebaucht sind.

6. Implantat nach einem der Ansprüche 2 bis 5, **dadurch gekennzeichnet, daß** die Vorsprünge (13) abgerundet sind.

7. Implantat nach einem der voranstehenden Ansprüche, **dadurch gekennzeichnet, daß** die Anlagefläche exzentrisch ist.

## Claims

1. Intervertebral implant comprising supporting surfaces adapted to abut on adjacent vertebral bodies (6), said surfaces being formed by the outer sides of two arms (2, 3) of the intervertebral implant, said arms being pivotable relative to one another, and a spreading member (8) arranged between the arms (2, 3) so as to be rotatable, said spreading member, during rotation, spreading the arms (2, 3) apart to different degrees by means of a contact surface, wherein the contact surface has at least one radial projection (13), during rotation of the spreading member (8) the arms (2, 3) being spread apart by means of said projection to a greater extent than in the two areas (12) located adjacent thereto in circumferential direction, wherein the spreading member (8) is adapted to be screwed into the intervertebral implant, **characterized in that** several projections (13) are distributed over the circumference of the spreading member (8) such that a polygonal cross section results and that the spreading member (8) is adapted to be screwed into a bore (7) with an internal thread arranged in the intervertebral implant.

2. Implant as defined in claim 1, **characterized in that** the projection (13) is formed by an edge between two adjacent circumferential areas (12) of the spreading member (8).

3. Implant as defined in claim 2, **characterized in that** the projection (13) extends essentially parallel to the axis of rotation of the spreading member (8).

4. Implant as defined in any one of the preceding claims, **characterized in that** the circumferential surfaces (12) between the projections (13) are essentially flat.

5. Implant as defined in any one of claims 1 to 3, **characterized in that** the circumferential surfaces (12) bulge in a spherical manner in the direction of the axis of rotation of the spreading member (8).

6. Implant as defined in any one of claims 2 to 5, **characterized in that** the projections (13) are rounded.

7. Implant as defined in any one of the preceding claims, **characterized in that** the contact surface is eccentric.

## Revendications

1. Implant intervertébral comportant des surfaces de support qui peuvent être appliquées contre des vertèbres (6) voisines, et qui sont formées par les côtés extérieurs de deux branches (2, 3) de l'implant intervertébral pouvant pivoter l'une par rapport à l'autre de manière opposée, et comprenant également un corps d'expansion (8) qui est agencé de manière rotative entre les branches (2, 3) et qui lors de la rotation écarte plus ou moins les branches (2, 3) au moyen d'une surface d'appui, la surface d'appui présentant au moins une protubérance radiale (13) par laquelle, lors de la rotation du corps d'expansion (8), les branches (2, 3) sont écartées davantage que dans les deux zones (12) situées de part et d'autre dans la direction périphérique, et le corps d'expansion (8) pouvant être vissé dans l'implant intervertébral,
**caractérisé en ce que** le long de la périphérie du corps d'expansion (8) sont réparties plusieurs protubérances (13) de manière à ce qu'il se forme une section transversale polygonale, et **en ce que** le corps d'expansion (8) peut être vissé dans un alésage à filetage intérieur (7) agencé dans l'implant intervertébral.

2. Implant selon la revendication 1, **caractérisé en ce que** la protubérance (13) est formée par une arête entre deux zones périphériques (12) voisines du corps d'expansion (8).

3. Implant selon la revendication 2, **caractérisé en ce que** la protubérance (13) s'étend sensiblement de manière parallèle à l'axe de rotation du corps d'expansion (8).

4. Implant selon l'une des revendications précédentes, **caractérisé en ce que** les surfaces périphériques (12) entre les protubérances (13) sont sensiblement planes.

5. Implant selon l'une des revendications 1 à 3, **caractérisé en ce que** les surfaces périphériques (12) sont creusées de manière bombée en direction de l'axe de rotation du corps d'expansion (8).

6. Implant selon l'une des revendications 2 à 5, **caractérisé en ce que** les protubérances (13) sont arrondies.

7. Implant selon l'une des revendications précédentes, **caractérisé en ce que** la surface d'appui est excentrée.
